# EUROPÄISCHE PATENTSCHRIFT

(11) **EP 1 393 774 B1**
(45) Veröffentlichungstag und Bekanntmachung des Hinweises auf die Patenterteilung: **27.06.2007**
(21) Anmeldenummer: 03090272.0
(22) Anmeldetag: 27.08.2003
(51) Int. Cl.: A61N 1/368, A61N 1/362

(54) **Herzschrittmacher**
Pacemaker
Stimulateur cardiaque

(30) Priorität: 02.09.2002 DE 10241089
(43) Veröffentlichungstag der Anmeldung: 03.03.2004
(73) Patentinhaber: Biotronik GmbH & Co. KG, 12359 Berlin (DE)
(72) Erfinder: Busch, Ulrich, 10318 Berlin (DE); Schaldach, Max, verstorben (DE)
(74) Vertreter: Lindner-Vogt, Karin L.

(56) Entgegenhaltungen:
- US-A- 5 584 867
- US-B1- 6 249 701

## Beschreibung

Die Erfindung betrifft einen biatrialen Dreikammer-Herzschrittmacher mit wenigstens einer Abtasteinheit für das Erfassen mit einer natürlichen Kontraktion eines Atriums und eines Ventrikels eines Herzens einhergehender Signale und mit wenigstens einer Stimulationseinheit, die zum Erzeugen von Stimulationsimpulsen für die Stimulation eines zweiten Atriums und des Ventrikels des Herzens ausgebildet ist. Der Herzschrittmacher umfasst außerdem eine Steuerung, die mit der Abtasteinheit und der Stimulationseinheit verbunden und ausgebildet ist, zumindest die dem ersten Atrium zugeordneten atrialen Senseereignisse und dem Ventrikel zugeordneten ventrikulären Senseereignisse für eine Ansteuerung der Stimulationseinheit auszuwerten. Die Ansteuerung erfolgt unter Berücksichtigung eines ventrikulären Escape-Intervalls und gegebenenfalls einer postatrialen ventrikulären Blanking-Zeit dergestalt, dass ein rechtsatriales Senseereignis das ventrikuläre Escape-Intervall auslöst, zu dessen Ende ein ventrikulärer Stimulationsimpuls ausgelöst wird, falls dieser nicht durch ein ventrikuläres Sense-Ereignis innerhalb des ventrikulären Escape-Intervalls und gegebenenfalls außerhalb der postatrialen ventrikulären Blanking-Zeit inhibiert wird. Die Ansteuerung erfolgt weiterhin unter Berücksichtigung einer interatrialen Überleitungszeit dergestalt, dass ein rechtsatriales Senseereignis die interatriale Überleitungszeit auslöst, zu deren Ende ein linksatrialer Stimulationsimpuls ausgelöst wird, der gegebenenfalls durch ein linksatriales Senseereignis innerhalb der atrialen Überleitungszeit inhibiert wird.

Derartige Dreikammer-Herzschrittmacher sind beispielsweise aus den US-Patenten 5,514,161 und 5,584,867 bekannt. Die beiden genannten US-Patente geben Lösungen an, mit denen ein Überleiten einer atrialen Tachykardie auf den Ventrikel eines Herzens vermittels des Herzschrittmachers verhindert werden kann.

Viele der an sich bekannten Zwei- und Dreikammer-Herzschrittmacher sind sogenannte Demand-Herzschrittmacher, die so ausgebildet sind, dass Stimulationsimpulse an eine Kammer eines Herzens nur dann ausgelöst werden, wenn innerhalb eines vorgegebenen Zeitfensters keine natürliche Herzaktion erfasst wird. Im Zusammenhang mit ventrikulären Herzschrittmachern spricht man beispielsweise von einem ventrikulären Escape-Intervall oder einer AV-Zeit, welche mit einer Stimulation oder einem natürlichen Ereignis im rechten Atrium des Herzens ausgelöst wird und zu deren Ende ein ventrikulärer Stimulationsimpuls erfolgt, sofern dieser nicht inhibiert wird, weil während des ventrikulären Escape-Intervalls ein Ereignis in dem zu stimulierenden Ventrikel des Herzens erfasst wurde.

Ein solches erfasstes Ereignis (V-Sense) kann auf eine natürliche Kontraktion des Ventrikels zurückgehen. Ähnliche elektrische Signale, wie sie bei der natürlichen Kontraktion eines Ventrikels entstehen, können jedoch auch auf ein Übersprechen von im Atrium eines Herzens erzeugten Signalen, beispielsweise einer atrialen Stimulation, auf den Ventrikel erzeugt werden. Um die Erfassung solcher aus dem Atrium stammender Signale im Ventrikel auszublenden, ist bei Schrittmachern üblicherweise eine Ausblendzeit (Blanking-Time) vorgesehen, die beispielsweise mit der Angabe eines Stimulationsimpulses an das rechte Atrium eines Herzens ausgelöst wird und zum Beispiel 100 ms dauert. Während dieser Ausblendzeit werden keine ventrikulären Sense-Ereignisse erfasst, so dass eventuell in dieser Ausblendzeit im Ventrikel auftretende Potentiale nicht zur Inhibierung der Abgabe eines ventrikulären Stimulationsimpulses führen.

Allgemein besteht der Wunsch, die Ausblendzeit so kurz wie möglich zu wählen, um zwar ein Übersprechen atrialer Stimulationsimpulse in den Abtastkanal für ventrikuläre Ereignisse zu verhindern, gleichzeitig jedoch natürliche ventrikuläre Ereignisse, insbesondere vorzeitige ventrikuläre Kontraktionen (PVC=premature ventricular contraction) zu detektieren. Das Erfassen und Berücksichtigen solcher vorzeitigen ventrikulären Kontraktionen ist auch deshalb gewünscht, damit diese die Abgabe eines ventrikulären Stimulationsimpulses unterdrücken können. Ansonsten könnte ein ventrikulärer Stimulationsimpuls im ungünstigsten Fall so ausgelöst werden, dass er in die sogenannte vulnerable Phase nach einer ventrikulären Kontraktion fällt.

Der skizzierten Problematik wird bei Zweikammer-Herzschrittmachern auf verschiedene Art und Weise begegnet. Genannt seien hier die US-Patente 5,776,167 und 4,825,870. Der Herzschrittmacher gemäß dem US-Patent 4,825,870 begegnet der zuvor skizzierten Problematik durch Vorsehen einer variablen Ausblendzeit und eines daran anschließenden Übersprechfensters (crosstalk window), während dessen ventrikuläre Ereignisse erfasst und als Übersprechereignisse (Crosstalk) gekennzeichnet werden.

Dem Stand der Technik ist bisher nicht zu entnehmen, dass bei einem Dreikammer-Herzschrittmacher ein Übersprechen aufgrund atrialer Stimulationen nicht nur auf eine Stimulation des rechten Atriums zurückgehen kann (wie dies bei Zweikammer-Herzschrittmachern bekannt ist), sondern auf eine Stimulation des linken Atriums. Der Erfindung setzt bereits bei der Erkenntnis an, dass die Stimulation des linken Atriums zeitlich nach der Stimulation des rechten Atriums erfolgt, und zwar nach einer atrialen Überleitungszeit, so dass ein Übersprechen vom linken Atrium zum (rechten) Ventrikel erst deutlich nach dem Übersprechen eines rechtsatrialen Stimulationsimpulses auftreten kann.

Dieser Problematik begegnet die Erfindung mit einem Herzschrittmacher der eingangs genannten Art, bei dem die Ansteuerung dergestalt erfolgt, dass die Abgabe eines atrialen Stimulationsimpulses unterdrückt wird, wenn entweder zuvor ein ventrikuläres Senseereignis in ein Übersprechzeitfenster (Crosstalk Window) fällt, welches sich an die postatriale ventrikuläre Ausblendzeit (Blanking-Time) anschließt, und gleichzeitig der zeitliche Abstand zum letzten, außerhalb eines Übersprech-Zeitfensters ermittelten ventrikulären Ereignisses bis zum nächstmöglichen ventrikulären Stimulationsereignis größer als ein vorgegebener Maximalwert ist und/oder wenn ein ventrikuläres Senseereignis während eines UTI (Upper tracking-Intervall)-Betriebsmodus auftritt, in dem der Herzschrittmacher in einer vorgegebenen, maximalen Stimulationsrate (UTR = Upper Tracking Rate) arbeitet.

Zusammengefasst besteht die Lösung des erfindungsgemäß erkannten Problems darin, die Abgabe eines linksatrialen Stimulationsimpulses und damit auch die Möglichkeit eines Übersprechens eines solchen Stimulationsimpulses auf den ventrikulären Abtastkanal ganz zu verhindern, falls ein ventrikuläres Ereignis auf Übersprechen wegen linksatrialer Stimulation zurückgehen könnte.

Da ein solches Übersprechen genau wie eine natürliche ventrikuläre Kontraktion eine Inhibierung des ventrikulären Stimulationsimpulses zur Folge haben müsste, könnte die ventrikuläre Stimulation im ungünstigsten Fall solange unterdrückt werden, dass dies physiologisch nachteilig ist.

In der ersten Variante wird daher die Abgabe eines linksatrialen Stimulationsimpulses immer genau dann unterdrückt, wenn eine dementsprechende Crosstalk-Wahrnehmung eine ventrikuläre Rate (gekennzeichnet durch ein länger als ein vorgegebenes maximales V-V-Intervall) zur Folge hätte, die physiologisch bedenklich ist.

Konkret misst ein Timer die jeweils seit dem letzten gesicherten ventrikulären Ereignis vergangene Zeit - unabhängig davon, ob das letzte ventrikuläre Ereignis eine natürliche Kontraktion oder eine Stimulation darstellt -und addiert zu dieser Zeit die bis zum nächsten geplanten ventrikulären Stimulationsimpuls noch vergehende Zeit hinzu. Es ergibt sich ein VV-Interval. Ist dieses aktuell berechnete VV-Intervall größer als ein vorgegebenes Maximal-Intervall, wird die nächstfolgende linksatriale Stimulation unterdrückt, um eine Fernfeldwahrnehmung des entsprechenden, links-atriale Stimulationsimpulses und eine hierdurch hervorgerufene Inhibierung des nächsten ventrikulären Stimulationsimpulses mit Sicherheit zu verhindern. Eine Inhibierung des nächsten geplanten ventrikulären Stimulationsimpulses aufgrund einer natürlichen ventrikulären Kontraktion ist dann immer noch möglich, auf Grund der Inhibierung des links-atriale Stimulationsimpulses kann die Inhibierung jedoch nicht auf einer Fernfeldwahrnehmung eines solchen Stimulationsimpulses zurückgehen.

In der alternativen Ausführungsvariante wird insbesondere berücksichtigt, dass bei Herzschrittmachern üblicherweise eine obere Grenze für die Stimulationsfrequenz vorgegeben ist. Überschreitet eine wahrgenommene atriale Rate diese obere Grenze für Stimulationsfrequenz, kann die Stimulation des Ventrikels nicht mehr atrium-synchron erfolgen. Im sogenannten Mode-Switching schaltet der Herzschrittmacher dann auf einen asynchronen Stimulationsmodus um, der mit einer oberen Abtastrate (upper tracking rate) einhergeht. Aufgrund der Asynchronizität atrialer und ventrikulärer Ereignisse ist die Inhibierung eines ventrikulären Stimulationsimpulses aufgrund einer Fernmeldewahrnehmung eines linksatriale Stimulationsimpulses gegeben. Daher wird die linksatriale Stimulation abgeschaltet, wenn der Schrittmacher in einem Betriebsmodus mit der größten vorgegebenen ventrikulären Stimulationsfrequenz arbeitet.

Die Erfindung soll nun anhand eines Ausführungsbeispiels mit Hilfe der Figuren näher erläutert werden. Von den Figuren zeigen
- Figur 1: eine Prinzipdarstellung eines Zweikammerherzschrittmachers
- Figur 2: ein Zeitdiagramm für die Steuerung eines Herzschrittmachers gemäß Figur 1

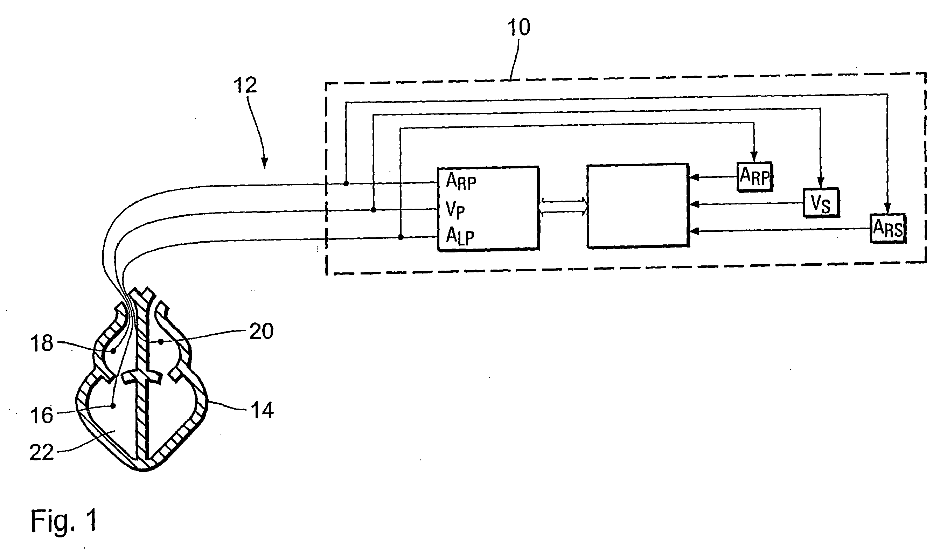

Üblicherweise ist ein Herzschrittmacher, wie er in Figur 1 mit der Bezugsziffer 10 angedeutet ist, mit einer Elektrodenleitung 12 verbunden, die in ein menschliches Herz 14 führt. Im Falle eines Dreikammerschrittmachers, wie dem in Figur 1 abgebildeten, sind Stimulations- und/ oder Abfühlelektroden 16 bis 20 so im Herzen 14 platziert, dass die Elektrode 16 elektrische Potenziale im linken Ventrikel 22 des Herzens 14 aufnimmt und Stimulationsimpulse an den linken Ventrikel 22 abgeben kann. Die Elektrode 16 kann uni- oder bipolar ausgebildet sein. Im erstgenannten unipolaren Fall weist die Elektrodenleitung 12 an ihrem in den linken Ventrikel führenden, distalen Ende eine einzige elektrisch leitende Oberfläche auf. Alternativ können auch zwei oder mehr elektrisch leitende Oberflächen vorgesehen sein, die es erlauben, Potenziale im linken Ventrikel zwischen zwei elektrisch leitenden Oberflächen aufzunehmen oder entsprechend Stimulationsimpulse abzugeben. Im unipolaren Fall bildet ein Gehäuse des Herzschrittmachers 10 jeweils den Gegenpol zu der ventrikulären Elektrode 16.

Entsprechendes gilt für die im rechten Atrium platzierte rechtsatriale Elektrode 18 und die im linken Atrium platzierte linksatriale Elektrode 20. Diese Elektroden können ebenfalls uni- oder bipolar ausgebildet sein. Elektrodenleitungen, die eine entsprechende Platzierung der Elektroden im rechten Ventrikel, dem rechten Atrium und dem linken Atrium erlauben, sind grundsätzlich bekannt.

Die Elektroden 16 bis 20 sind über jeweils separate elektrische Leitungen der Elektrodenleitung 12 mit dem Herzschrittmacher 10 verbunden. Es ist auch denkbar, dass anstelle separater elektrischer Leitungen in der Elektrodenleitung 12 weniger elektrische Leitungen als Elektrodenoberflächen vorgesehen sind, die dann beispielsweise im multiplexing-Verfahren von den Elektroden im Herzen aufgenommene elektrische Potenziale als Signale zum Herzschrittmacher leiten oder umgekehrt Stimulationsimpulse vom Herzschrittmacher zum Herzen leiten.

Der Herzschrittmacher 10 ist derart ausgebildet, dass er mittels einer Stimulationseinheit 30 wahlweise und gesteuert elektrische Stimulationsimpulse über die Elektrodenleitung 12 und eine oder mehrere der Elektroden 16 bis 20 an die entsprechende Kammer des Herzens 14 abgeben kann. Im Falle eines reinen Schrittmacherbetriebes, der die zeitgerechte Stimulation der einzelnen Kammer für eine bedarfsgerechte Blutversorgung eines Patienten dient, wird zu einem Zeitpunkt jeweils nur eine der Kammern stimuliert.

Für eine entsprechende Ansteuerung ist die Stimulationseinheit 30 mit einer Steuereinheit 32 verbunden, die für eine solche, bedarfsgerechte Ansteuerung der Stimulationseinheit 30 und entsprechend eine Stimulationsimpulsabgabe an eine der Kammern des Herzens 14 sorgt.

Eine Stimulation einer jeweiligen Kammer soll eine Kontraktion der jeweiligen Kammer zur Folge haben. Die Erzeugung entsprechend geeigneter Stimulationsimpulse sowie auch die Kontrolle des Stimulationserfolges sind grundsätzlich bekannt.

Die zeitliche Abfolge der Stimulationsimpulse ist so gewählt, dass sich eine Zeitabfolge der Kontraktionen der Herzkammern ergibt, wie sie bei einem gesunden Herzen auf natürliche Weise auftritt. Auf eine Kontraktion des rechten Atriums folgt nach einer atrio-ventrikulären Überleitungszeit (AV-Überleitungszeit) eine Kontraktion des rechten Ventrikels. Ebenfalls ausgehend von einer Kontraktion des rechten Atriums erfolgt zeitversetzt die Kontraktion des linken Atriums nach einer interatrialen Überleitungszeit (AA-Überleitungszeit).

Auf die Kontraktion des rechten Ventrikels erfolgt nach einer VA-Zeit wiederum eine Kontraktion des rechten Atriums. Die Kontraktion der einzelnen Herzkammern geht mit einer Depolarisierung des Herzgewebes (Myokards) einher, welche zu messbaren elektrischen Potenzialen führt. Auf die Depolarisierung und Kontraktion des Myokards erfolgt eine Repolarisierung, nach der das Myokard zu einer erneuten Kontraktion bereit ist.

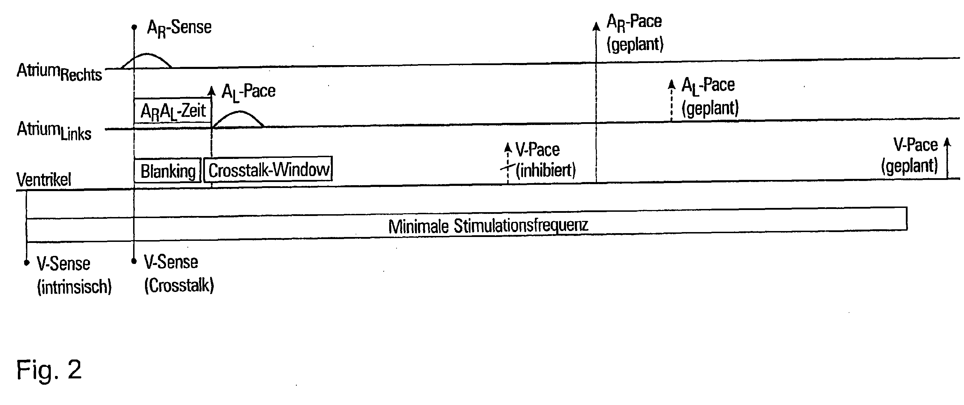

Die Steuereinheit 32 ist daher so ausgebildet, dass sie auf ein erfasstes natürliches Ereignis im rechten Atrium (A_{R}-Sense) einen A-A-Intervall-Timer startet, falls innerhalb der durch diesen Timer vorgegebenen A_{R}A_{L}-Zeit eine natürliche Kontraktion des linken Atriums erfasst wird (A_{L}-Sense) wird eine Stimulation des linken Atriums (A_{L}-Pace) am Ende der A_{R}A_{L}-Zeit inhibiert. Falls während der A_{R}A_{L}-Zeit kein A_{L}-Sense erfasst wird, wird am Ende der A_{R}A_{L}-Zeit ein linksatrialer Stimulationsimpuls abgegeben; siehe Figur 2.

Ebenfalls mit einem rechtsatrialen Ereignis, sei es das Erfassen einer natürlichen rechtsatrialen Kontraktion (A_{R}-Sense) oder die Abgabe eines rechtsatrialen Stimulationsimpulses (A_{R}-Pace), wird ein ventrikuläres Escape-Intervall ausgelöst, an dessen Ende ein (rechts-)ventrikulärer Stimulationsimpuls (V-Pace) abgegeben wird, falls innerhalb des Escape-Intervalls keine natürliche Kontraktion des Ventrikels (V-Sense) erfasst wird. Wird innerhalb des Escape-Intervalls ein möglicherweise eine ventrikuläre Kontraktion kennzeichnendes elektrisches Potential erfasst, wird der ventrikuläre Stimulationsimpuls (V-Pace) am Ende des Escape-Intervalls inhibiert (siehe Figur 2). Die Dauer des Escape-Intervalls hängt bei einem ratengesteuerten Schrittmacher in der Regel von einem den physiologischen Bedarf eines Patienten kennzeichnenden Messwert ab.

Das rechtsatriale Ereignis (A_{R}-Sense oder A_{R}-Pace) löst nicht nur das ventrikuläre Escape-Intervall (auch AV-Verzögerung genannt) aus, sondern auch zunächst ein Blanking-Intervall von beispielsweise hundert Millisekunden Dauer, innerhalb dessen die Steuerung 32 keine ventrikulären Sense-Ereignisse (V-Sense) erfasst, oder diese zumindest nicht verarbeitet. Im ersten Fall spricht man von absoluter Refraktärzeit des Schrittmachers, im zweiten Fall von relativer Refraktärzeit. Das Blanking-Invervall hat wie eingangs erläutert den Zweck einer Fernfeldwahrnehmung eines Stimulationsimpulses für das rechte Atrium im rechten Ventrikel auszublenden, damit solch ein auf Fernfeldwirkung beruhendes Potential im rechten Ventrikel nicht erfasst wird und zur Inhibierung eines ventrikulären Stimulationsimpulses führt.

Der in Figur 1 dargestellte Dreikammerschrittmacher bringt weiterhin die Möglichkeit mit sich, auch das linke Atrium zu stimulieren. In Figur 2 ist die Abgabe eines linksatrialen Stimulationsimpulses (A_{L}-Pace) nach Ablauf der A_{R}A_{L}-Zeit dargestellt. Auch der linksatriale Stimulationsimpuls kann mittels Fernfeldwahrnehmung zu einem ventrikulären Abtastereignis (V-Sense) führen, wie dies in Figur 2 durch eine gestrichelte Linie ebenfalls dargestellt ist. Diese Wahrnehmung liegt außerhalb der Ausblendzeit und führt daher zu einer Inhibierung des ansonsten am Ende des ventrikulären Escape-Intervalls abgegebenen ventrikulären Stimulationsimpulses (V-Pace). Hintergrund ist, dass eine vorzeitige natürliche Kontraktion des Ventrikels (PVC = premature ventricular contraction) in der Tat eine Inhibierung des ventrikulären Stimulationsimpulses zur Folge haben soll. Auf Grund der Inhibierung des ventrikulären Stimulationsimpulses wegen der Fernfeldwahrnehmung des linksatrialen Stimulationsimpulses kann der nächste ventrikuläre Stimulationsimpuls frühestens nach Ablauf eines weiteren ventrikulären Escape-Intervalls erfolgen, welches durch ein rechtsatriales Ereignis ausgelöst wird, dass nach einer VA-Zeit auf den inhibierten ventrikulären Stimulationsimpuls folgt. Auch der nächste bereits getimte ventrikuläre Stimulationsimpuls (in Figur 2 als V-Pace (geplant) eingezeichnet) könnte bei einem herkömmlichen Dreikammerschrittmacher wiederum durch einen linksatrialen Stimulationsimpuls inhibiert werden. Auf diese Weise kann es vorkommen, dass der Ventrikel für einige Perioden nicht stimuliert wird und in dieser Zeit möglicherweise auch nicht auf natürliche Art kontrahiert.

Daher ist die Steuereinheit des hier beschriebenen erfindungsgemäßen Dreikammerschrittmachers so ausgelegt, dass mit dem letzten sicheren ventrikulären Ereignis - sei es ein intrinsisches, natürliches Ereignis oder auch ein stimuliertes Ereignis - ein Zeitzähler gestartet wird und regelmäßig verglichen wird, ob die Zeit von dem letzten sicheren ventrikulären Ereignis bis zum nächsten geplanten Ereignis ein vorgegebenes Maximum überschreitet. Dieses vorgegebene Maximum ist vorzugsweise in einem nichtflüchtigen Speicher des Herzschrittmachers abgelegt und kann entweder durch einen Arzt vorgebbar sein oder aber auch selbstjustierend eingestellt werden.

Weiterhin ist die Steuereinheit 32 derart ausgestaltet, dass sie ein mit Ende der Ausblendzeit nach einem rechtsatrialen Ereignis beginnendes Übersprechfenster (Crosstalk-Window) berücksichtigt und im Falle ventrikulärer Ereignisse - wie beispielsweise der Fernfeldwahrnehmung des linksatrialen Stimulationsimpulses - innerhalb des Übersprechfensters prüft, ob der nächste geplante ventrikuläre Stimulationsimpuls die zuvor beschriebene Überschreitung einer maximalen V-V-Zeitdauer zur Folge hätte (dies entspricht einer minimalen Stimulationsfrequenz). Falls die beiden Bedingungen, nämlich einerseits ein ventrikuläres Ereignis im Übersprechfenster und zum anderen eine mögliche Überschreitung einer maximalen V-V-Zeitdauer, erfüllt sind, löst die Steuereinheit 32 mit dem nächstfolgenden rechtsatrialen Ereignis nicht die interatriale Überleitungszeit (A_{R}A_{L}-Zeit) aus, zu deren Ende ein linksatrialer Stimulationsimpuls abgegeben würde. De facto schaltet die Steuereinheit den Dreikammerschrittmacher auf einen Zweikammerbetrieb um. Damit wird sichergestellt, dass kein linksatrialer Stimulationsimpuls ausgegeben wird, der die Inhibierung eines ventrikulären Stimulationsimpulses mit der Folge bewirken würde, dass das resultierende V-V-Intervall die vorgegebene Maximaldauer überschreitet.

In einer vereinfachten Ausführungsform ist eine alternative Steuereinheit 32 so ausgebildet, dass die interatriale Synchronisation immer dann abgeschaltet wird - also das Umschalten auf den Zweikammermodus immer dann erfolgt - wenn ein ventrikuläres Sense-Ereignis erfasst wird, während sich der Herzschrittmacher in einer Betriebsart befindet, in der er mit einer maximal vorgegebenen Stimulationsrate (Upper Tracking Rate) arbeitet. In diesem Fall ist die Stimulation des Ventrikels nicht mehr durch atriale Ereignisse gesteuert, da diese atrialen Ereignisse eine zu hohe ventrikuläre Rate zur Folge hätten. Auf dem Wege des an sich bekannten Mode-Switchings schaltet die alternative Steuereinheit 32 den Herzschrittmacher vom atriumsynchronen Modus in einen asynchronen Modus um, in dem der Ventrikel mit der maximalen Stimulationsrate stimuliert wird. Auf Grund dieser Asynchronizität könnten linksatriale Stimulationsimpulse nach Ablauf einer intraatrialen Überleitungszeit leicht vermittels Fernfeldwahrnehmung zu ventrikulären Senseereignissen führen, die nicht auf eine natürliche Kontraktion des Ventrikels zurückgehen. Es könnte zu der zuvor beschriebenen länger dauernden Unterdrückung der Stimulation des Ventrikels kommen. Daher ist die alternative Stimulationseinheit 32 so ausgebildet, dass sie im Falle eines ventrikulären Senseereignisses im Upper Tracking Rate-Modus die interatriale Synchronisation abschaltet.

## Patentansprüche

1. Biatrialer Dreikammer-Her-zschrittmacher mit
- wenigstens einer Abtasteinheit für Senseereignisse eines ersten Atriums und eines Ventrikels eines Herzens und
- wenigstens einer Stimulationseinheit, die zum Erzeugen von Stimulationsimpulsen an ein zweites Atrium und an den Ventrikel ausgebildet ist, sowie
- einer Steuerung,
- die mit der Abtasteinheit und der Stimulationseinheit verbunden und
- ausgebildet ist, zumindest die dem ersten Atrium zugeordneten atrialen Senseereignisse (A_{R}-Sense) und dem Ventrikel zugeordneten ventrikulären Senseereignisse (V-Sense) für eine Ansteuerung der Stimulationseinheit auszuwerten,
- wobei die Ansteuerung unter Berücksichtigung eines ventrikulären Escape-Intervalls und einer postatrialen ventrikulären Ausblend-Zeit (Blanking) dergestalt erfolgt, dass ein rechtsatriales Senseereignis (A_{R}-Sense) das ventrikuläre Escape-Intervall auslöst, zu dessen Ende ein ventrikulärer Stimulationsimpuls ausgelöst wird, falls dieser nicht durch ein ventrikuläres Sense-Ereignis innerhalb des ventrikulären Escape-Intervalls und außerhalb der postatrialen ventrikulären Blanking-Zeit inhibiert wird,
- wobei die Ansteuerung weiterhin unter Berücksichtigung einer interatrialen Überleitungszeit dergestalt erfolgt, dass ein rechtsatriales Senseereignis (A_{R}-Sense) die interatriale Überleitungszeit auslöst, zu deren Ende ein linksatrialer Stimulationsimpuls ausgelöst wird, falls der linksatriale Stimulationsimpuls nicht inhibiert wird,
**dadurch gekennzeichnet, dass** die Ansteuerung weiterhin dergestalt erfolgt, dass die Abgabe eines linksatrialen Stimulationsimpulses unterdrückt wird,
- wenn zuvor ein ventrikuläres Sense-Ereignis in ein Übersprech-Zeitfenster, Crosstalk-Window, fällt, welches sich an eine postatriale ventrikuläre Ausblend-Zeit, Blanking, anschließt, und gleichzeitig der zeitliche Abstand zum letzten, außerhalb eines Übersprech-Zeitfensters ermittelten ventrikulären Ereignisses bis zum nächstmöglichen ventrikulären Stimulationsereignis größer als ein vorgegebener Maximal-Wert ist.

2. Biatrialer Dreikammer-Herzschrittmacher nach dem Oberbegriff von Anspruch 1, **dadurch gekennzeichnet, dass** die Ansteuerung weiterhin dergestalt erfolgt, dass die Abgabe eines linksatrialen Stimulationsimpulses unterdrückt wird,
- wenn ein ventrikuläres Senseereignis während eines UTI-Betriebsmodus auftritt, in dem der Herzschrittmacher mit einer vorgegeben, maximalen Stimulationsrate arbeitet.

3. Biatrialer Dreikammer-Herzschrittmacher nach Anspruch 1 und/oder 2, mit einer Abtasteinheit für Senseereignisse eines zweiten (linken) Atriums eines Herzens **dadurch gekennzeichnet, dass** die Ansteuerung weiterhin dergestalt erfolgt, dass die Abgabe eines linksatrialen Stimulationsimpulses unterdrückt wird, falls die Abtasteinheit ein für ein linksatriales Sense-Ereignis (A_{L}-Sense) innerhalb der interatrialen Überleitungszeit charakteristisches Signal erzeugt.

4. Biatrialer Dreikammer-Herzschrittmacher nach einem der Ansprüche 1 bis 3, **gekennzeichnet durch** eine Steuereinheit, die zum Berechnen des Zeitabstandes von einem jüngsten gesicherten ventrikulären Ereignis bis zu einem nächsten geplanten ventrikulären Stimulationsimpuls ausgebildet ist.

5. Biatrialer Dreikammer-Herzschrittmacher nach Anspruch 4, **gekennzeichnet durch** eine Steuereinheit, die zum Vergleichen des errechneten Zeitabstandes mit einem vorgebbaren Maximalwert ausgebildet ist.

6. Biatrialer Dreikammer-Herzschrittmacher nach Anspruch 5, **gekennzeichnet durch** eine Steuereinheit, die zum Abschalten einer interatrialen Sychronisation in Abhängigkeit des Vergleiches zwischen errechnetem Zeitabstand und vorgegebenem Maximalwert ausgebildet ist.

## Claims

1. A biatrial triple-chamber cardiac pacemaker having
- at least one tracking unit for sense events of a first atrium and a ventricle of a heart and
- at least one stimulation unit, which is implemented to generate stimulation pulses at a second atrium and at the ventricle, and
- a controller,
- which is connected to the tracking unit and the stimulation unit and
- is implemented to analyze at least the atrial sense events (A_{R} sense) assigned to the first atrium and the ventricular sense events (V sense) assigned to the ventricle for an activation of the stimulation unit,
- the activation being performed in consideration of a ventricular escape interval and a post-atrial ventricular blanking time in such a way that a right-atrial sense event (A_{R} sense) triggers the ventricular escape interval, at the end of which a ventricular stimulation pulse is triggered, if this is not inhibited by a ventricular sense event within the ventricular escape interval and outside the post-atrial ventricular blanking time,
- the activation also being performed in consideration of an interatrial transfer time in such a way that a right-atrial sense event (A_{R} sense) triggers the interatrial transfer time, at the end of which a left-atrial stimulation pulse is triggered, if the left-atrial stimulation pulse is not inhibited,
**characterized in that** the activation is also performed in such a way that the delivery of a left-atrial stimulation pulse is suppressed,
- if a ventricular sense event previously falls in a crosstalk window, which follows a post-atrial ventricular blanking time, and simultaneously the chronological interval to the last ventricular event ascertained outside a crosstalk time window up to the next possible ventricular stimulation event is greater than a predefined maximum value.

2. The biatrial triple-chamber cardiac pacemaker according to the preamble of Claim 1, **characterized in that** the activation is also performed in such a way that the delivery of a left-atrial stimulation pulse is suppressed,
- if a ventricular sense event occurs during a UTI operating mode, in which the cardiac pacemaker operates at a predefined, maximum stimulation rate.

3. The biatrial triple-chamber cardiac pacemaker according to Claim 1 and/or 2, having a tracking unit for sense events of a second (left) atrium of a heart, **characterized in that** the activation is also performed in such a way that the delivery of a left-atrial stimulation pulse is suppressed if the tracking unit generates a signal characteristic for a left-atrial sense event (A_{L} sense) within the interatrial transfer time.

4. The biatrial triple-chamber cardiac pacemaker according to one of Claims 1 through 3, **characterized by** a control unit, which is implemented to calculate the time interval from a most recent confirmed ventricular event up to a next planned ventricular stimulation pulse.

5. The biatrial triple-chamber cardiac pacemaker according to Claim 4, **characterized by** a control unit, which is implemented to compare the calculated time interval to a predefinable maximum value.

6. The biatrial triple-chamber cardiac pacemaker according to Claim 5, **characterized by** a control unit, which is implemented to shut down an interatrial synchronization as a function of the comparison between calculated time interval and predefined maximum value.

## Revendications

1. Pacemaker à trois chambres biatrial comprenant
- au moins une unité de balayage pour des événements de "sense" d'un premier atrium et d'un ventricule d'un coeur et
- au moins une unité de stimulation, qui est conçue pour générer des impulsions de stimulation sur un second atrium et sur le ventricule, et
- une commande,
- qui est reliée à l'unité de balayage et à l'unité de stimulation et
- est conçue pour analyser au moins les événements de "sense" (A_{R}-Sense) atriaux attribués au premier atrium et des événements de "sense" (V-Sense) ventriculaires attribués au ventricule pour une activation de l'unité de stimulation,
- l'activation s'effectue en tenant compte d'un intervalle de "escape" ventriculaire et d'un temps de suppression (blanking) ventriculaire postatrial de telle sorte qu'un événement de "sense" (A_{R}-Sense) atrial droit déclenche l'intervalle de "escape" ventriculaire, à la fin duquel une impulsion de stimulation ventriculaire est déclenchée lorsque celle-ci n'est pas inhibée par un événement de "sense" ventriculaire à l'intérieur de l'intervalle de "escape" ventriculaire et à l'extérieur du temps de "blanking" ventriculaire postatrial,
- l'activation s'effectuant également en tenant compte d'un temps de transfert interatrial de telle sorte qu'un événement de sense atrial droit (A_{R}-Sense) déclenche le temps de transfert interatrial, à la fin duquel une impulsion de stimulation atriale gauche est déclenchée, lorsque l'impulsion de stimulation atriale gauche n'est pas inhibée,
**caractérisé en ce que** l'activation s'effectue de telle sorte que l'envoi d'une impulsion de stimulation atriale gauche est supprimé,
- lorsque auparavant, un événement de "sense" ventriculaire tombe dans une fenêtre de temps de diaphonie, Crosstalk-Window, qui fait suite à un temps de suppression, Blanking, ventriculaire postatrial, et dans le même temps, l'intervalle de temps par rapport au dernier événement ventriculaire déterminé à l'extérieur d'une fenêtre de temps de diaphonie jusqu'au prochain événement de stimulation ventriculaire est supérieur à une valeur maximale prédéfinie.

2. Pacemaker à trois chambres biatrial selon le préambule de la revendication 1, **caractérisé en ce que** l'activation s'effectue de telle sorte que l'envoi d'une impulsion de stimulation atriale gauche est supprimé,
- lorsqu'un événement de "sense" ventriculaire apparaît pendant un mode de service UTI, dans lequel le pacemaker travaille avec un taux de stimulation maximum prédéfini.

3. Pacemaker à trois chambres biatrial selon la revendication 1 et/ou 2, comprenant une unité de balayage pour les événements de "sense" d'un second atrium (gauche) d'un coeur, **caractérisé en ce que** l'activation s'effectue de telle sorte que l'envoi d'une impulsion de stimulation atriale gauche est supprimé dans le cas où l'unité de balayage génère un signal caractéristique d'un événement de "sense" atrial gauche (A_{L}-Sense) à l'intérieur du temps de transfert interatrial.

4. Pacemaker à trois chambres biatrial selon l'une quelconque des revendications 1 à 3, **caractérisé par** une unité de commande, qui est conçue pour calculer l'intervalle de temps entre un événement ventriculaire sécurisé très récent et une prochaine impulsion de stimulation ventriculaire planifiée.

5. Pacemaker à trois chambres biatrial selon la revendication 4, **caractérisé par** une unité de commande, qui est conçue pour comparer l'intervalle de temps calculé avec une valeur maximale prédéfinissable.

6. Pacemaker à trois chambres biatrial selon la revendication 5, **caractérisé par** une unité de commande, qui est conçue pour déconnecter une synchronisation interatriale en fonction de la comparaison entre l'intervalle de temps calculé et la valeur maximale prédéfinie.
